# EUROPEAN PATENT APPLICATION

(11) **EP 4 083 027 A1**
(43) Date of publication of application: **02.11.2022**
(21) Application number: 21382362.8
(22) Date of filing: 26.04.2021
(51) Int. Cl.: C07D 263/57

(54) **A SOLID STATE FORM OF TAFAMIDIS AND A PROCESS FOR ITS PREPARATION**

(71) Applicant: Química Sintética, S.A., 28805 Alcalá de Henares (ES)
(72) Inventor: BARRECA, Giuseppe, Sirtori (IT); CARCONE, Luca, Milan (IT); PARAVIDINO, Piero, Sedriano (IT); PENGO, Daniele, Senago (IT); ZAMPIERI, Massimo, Cesano Maderno (IT); LIMING, Huang, Hangzhou City (CN)
(74) Representative: Aera A/S

(57) **Abstract**

A crystalline form of 2-(3,5-dichlorophenyl)-1,3-benzoxazole-6-carboxylic acid and a method for its preparation are described.

## Description

### Field of the invention

The present invention relates to a crystalline form of tafamidis and to a method for its preparation.

### Background art

2-(3,5-dichlorophenyl)-1,3-benzoxazole-6-carboxylic acid (tafamidis) is a drug used to delay loss of peripheral nerve function in adults with familial amyloid polyneuropathy, a fatal, although relatively rare disease linked to a hereditary mutation of the transthyretin (TTR) gene, and for the treatment of heart disease (cardiomyopathy) caused by transthyretin-mediated amyloidosis (ATTR-CM).

In May 2019, the FDA approved two separate preparations for oral administration, one containing Tafamidis (Vyndamax^{®}) and one containing Tafamidis Meglumine (Vyndaqel^{®}), for the treatment of the cardiomyopathy of wild-type or hereditary transthyretin-mediated amyloidosis (ATTR-CM) in adults to reduce cardiovascular mortality and cardiovascular-related hospitalization.

As generally known, an active principle may exist under amorphous or different crystalline forms (polymorphs), either as pure compound or in forms in which molecules of water (hydrates) or of another solvent (solvates) are present in the structure of the crystal. In case of hydrates and solvates, the ratio between the number of molecules of active principle and molecules of water or solvent may vary, giving rise to different solid forms of the compound.

In particular, amorphous solids consist of disordered arrangement of molecules and do not possess a distinguishable crystal lattice.

A specific solid form of a compound may possess physical properties that differ from, and are advantageous over, those of other crystalline modifications. These include, but are not limited to, packing properties such as molar volume and density, thermodynamic properties such as melting point and glass transition temperature and solubility, kinetic properties such as dissolution rate, surface properties such as wettability interfacial tension, handling and filtration properties. Variations in any of these properties may affect the chemical and pharmaceutical processing of a compound and may often render a specific solid form more suitable than others for pharmaceutical and medical use.

2-(3,5-dichlorophenyl)-1,3-benzoxazole-6-carboxylic acid was first described in WO 2004/056315A2 (filed on 19 December 2003), wherein tafamidis is disclosed as compound (19) and obtained as a white solid, by final isolation of the acid via preparative TLC.

WO 2016/38500 discloses various crystalline forms of Tafamidis, namely forms 1, form 2 (a THF solvate), form 4, form 6 and an amorphous form.

As used herein, *"tafamidis form 4"* refers to the form described in WO 2016/38500 and characterized by a powder X-ray diffraction pattern comprising peaks at diffraction angles (2-theta) of 15.9, 16.9 and, optionally, also 18.0 and 27.3 (all ± 0.2, using Cu Kα1 radiation).

As used herein, *"tafamidis form 1*" refers to the form described in WO 2016/38500 and characterized by a powder X-ray diffraction pattern comprising the peaks at diffraction angle (2-theta) of 28.6 and, optionally, also the peaks at 15.4, 16.5, 20.2 and 29.0 2-theta (all ± 0.2, using Cu Kα1 radiation).

WO 2013/038351 describes the N-methyl-D-glucamine (meglumine) salt of tafamidis, specifically the anhydrous form M having a powder X-ray diffraction pattern comprising peaks at diffraction angles (2-theta) of 10.7, 11.8 and 13.3 (all ± 0.2, using Cu Kα₁ radiation), that is the solid active product ingredient form present in the medicinal product Vyndaqel according to the EPAR- public assessment report issued by the European Medicine Agency (EMA) in date 22 September 2011.

The article published on Angewandte Chemie Int. Ed. 2003, 42, 2758-2761 describes a method for obtaining tafamidis by concentration in vacuo of flash column chromatography fractions (4.9 : 95 : 0.1 MeOH : CH₂Cl₂: AcOH). According to the information provided during the examination of EP 3191461 (deriving from the above mentioned WO 2016/38500), the thus-obtained solid is amorphous and, upon storage for 1 week at 70 °C/75% relative humidity, it converts into crystalline form 4.

Various solid forms of tafamidis, including adducts with formic acid, trifluoroacetic acid and acetic acid, have been described in WO 2019/175263.

WO 2020/232325 is relative to tafamidis and describes an amorphous form, form I (hydrate form, obtained by exposure of the amorphous form to water vapours for 30-33 days or from THF/water), form II (from 2-methylTHF, obtained by crash cooling or by slow evaporation of solvent over 7 days), forms III and IV (solvate with acetic acid) and form V (anhydrous or solvated with methanol). Forms I, II, III and IV convert into form 4 when dried in vacuum dryer at 100-160°C.

WO 2021/019448 discloses a process for the preparation of Tafamidis meglumine without isolation of Tafamidis in the free acid form and reports the XRPD pattern of the intermediate 4-[(3,5-dichlorobenzoyl)amino]-3-hydroxybenzoic acid, obtained by the procedure disclosed in WO 2016/38500.

The methods for the preparation of forms 1 and 4 of tafamidis and of form M of tafamidis meglumine described in the documents cited above are relative to laboratory scale trials (10 mg to 1-1.5 grams) and are unsuitable for the production on large scale due to the volumes of solvents used and/or the exceeding long time required. An object of this invention is, therefore, to provide crystalline forms of tafamidis which are chemically and polymorphically stable, suitable to be produced on large scale and to be converted into other crystalline forms or incorporated as such in a pharmaceutical dosage form.

### Summary of the invention

These objectives are achieved with the present invention that, in an aspect thereof, relates to a polymorphically and/or chemically stable crystalline form of 2-(3,5-dichlorophenyl)-1,3-benzoxazole-6-carboxylic acid (tafamidis), preferably a polymorphically and chemically stable crystalline form of tafamidis (hereafter also referred to as crystalline form alpha), said stable crystalline form being characterized by an XRPD profile comprising at least one of the peaks at 6.4, 11.4, 13.5, 16.3, 18.2, 20.4 and 27.5 degrees 2θ (± 0.2), when collected with the Kα radiation of copper (λ = 1.5418 Å), or a combination thereof In another aspect, the present invention relates to a process for the preparation of the crystalline form alpha of 2-(3,5-dichlorophenyl)-1,3-benzoxazole-6-carboxylic acid, comprising the steps of:
i. providing a solution of 2-(3,5-dichlorophenyl)-1,3-benzoxazole-6-carboxylic acid in a solvent;
ii. adding the solution of step i. to an anti-solvent:
iii. isolating the obtained solid.

In another aspect, the invention relates to the use of form alpha of 2-(3,5-dichlorophenyl)-1,3-benzoxazole-6-carboxylic acid for the preparation of a solid form of 2-(3,5-dichlorophenyl)-1,3-benzoxazole-6-carboxylic acid other than form alpha and a salt or adduct thereof.

In another aspect, the present invention is relative to a pharmaceutical formulation comprising form alpha of 2-(3,5-dichlorophenyl)-1,3-benzoxazole-6-carboxylic acid as described above.

### Brief description of the drawings

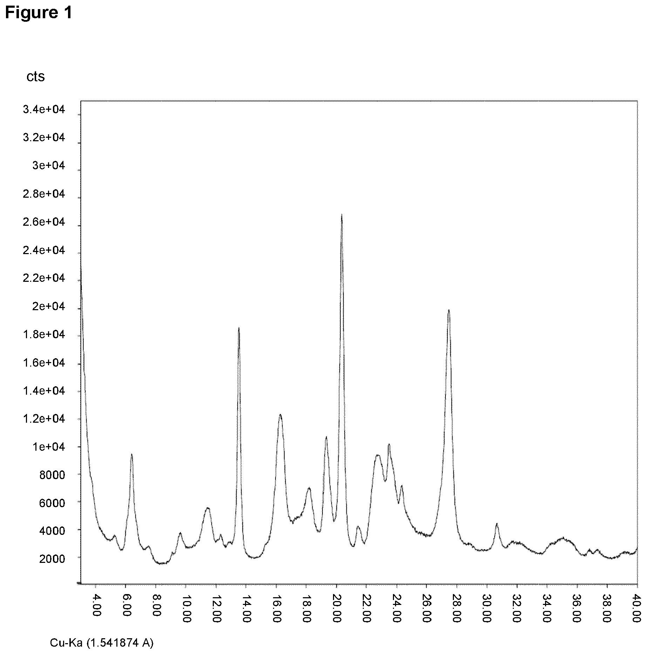

Figure 1 depicts an exemplary X-ray powder diffractogram (XRPD) of the solid form alpha of 2-(3,5-dichlorophenyl)-1,3-benzoxazole-6-carboxylic acid.

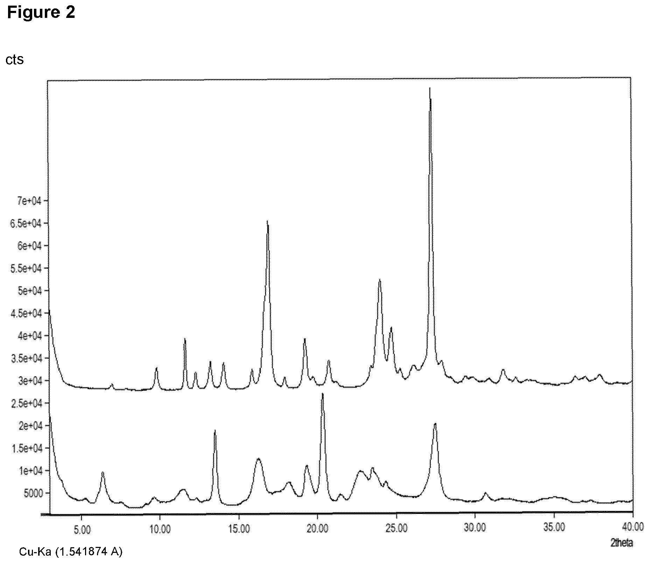

Figure 2 shows a comparison of the XRPD patterns of form alpha (lower curve) and of form 4 (upper curve) of 2-(3,5-dichlorophenyl)-1 ,3-benzoxazole-6-carboxylic acid.
Figure 3 shows the differential scan calorimetry (DSC) curve of the solid form alpha of 2-(3,5-dichlorophenyl)-1,3-benzoxazole-6-carboxylic acid (11 mg; 30 - 300 °C; 10 °C/min)
Figure 4 shows the thermogravimetric analysis (TGA) curve of the solid form alpha of 2-(3,5-dichlorophenyl)-1,3-benzoxazole-6-carboxylic acid (5 °C/min, 30-300 °C).

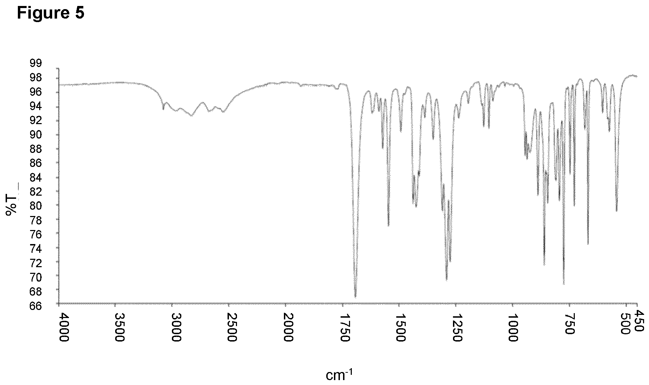

Figure 5 shows the infrared (IR) spectrum of the solid form alpha of 2-(3,5-dichlorophenyl)-1,3-benzoxazole-6-carboxylic acid.

### Detailed description of the invention

All terms used in this application, unless otherwise specified, are to be understood in their ordinary meaning as known in the technical field.

The term *"about"* includes the range of experimental errors, which can normally occur performing a measurement, e.g. ± 5% or ±2% or ±1%.

The term "mass" defines the combination of substrates, reagents, solvents, and products on which a physical or chemical transformation is carried out.

The term *"excipient"* means any substance contained in the final pharmaceutical form other than the active ingredient and which generally may not be therapeutically effective by itself. Excipients are essential for the administration of the active substance, as they allow to deliver the drug to the target site. Excipients are commonly referred to as raw materials entering into the composition of a pharmaceutical preparation with the aim of giving a shape, to facilitate administration and preserve the active ingredient. Furthermore, they contribute to characterize the pharmaceutical preparation from the point of view of appearance, stability, biopharmaceutical profile and acceptability by the patient.

Unless otherwise indicated, in the context of the present invention the percentage and amount of a certain component in a composition are to be referred to the weight of said component with respect to the total weight of the composition.

Unless otherwise indicated, in the context of the present invention the indication that a composition *"comprises"* other one or more components/elements means that the indicated components/elements must be present and also other components may be present, but are not necessarily present, in the composition, in addition to the ones specifically recited. In other words, the indication that a composition *"comprises"* one or more components does not exclude that the composition *consists* of, or consists essentially of, the recited component(s).

As used herein, the indication that a compound or composition A is *"pure"* or *"entirely free"* of other substances (or "*consists of"*) means that, within the detection range of the instrument or method being used, no substances other than those specifically indicated can be detected in A.

As used herein, the term "a *compound or composition A is essentially free of other substance(s)",* or *"consists essentially of A",* means that only trace amount of substance(s) other than A, if any, can be detected using the analytical methods and techniques known to the person skilled in the art.

Unless otherwise indicated, in the context of the present invention a range of values indicated for a certain parameter, for example the weight of a component in a mixture, includes the upper and the lower limits of the range, *e.g.* if the content in weight, or in volume, of a component A in a mixture is indicated as *"X to Y",* the content of A can be X, Y or any of the intermediate values.

By *"polymorphically stable"* it is meant that the crystalline form of the present invention, when stored (I) at 70 °C under reduced pressure for at least 1 hour (preferably for 5 hours, more preferably for 10 hours, even more preferably for 12 hours), (II) at 60 °C for at least 1 day (preferably for 5 days, more preferably for 10 days, even more preferably for 15 days), (III) at 40 °C and 75% RH for at least 1 day (preferably for 5 days, more preferably for 10 days, even more preferably for 15 days), and/or (IV) at 25-30 °C and 80% RH for at least 1 day (preferably for 5 days, more preferably for 10 days, even more preferably for 15 days), shows no signs of transformation into a different crystalline form as evaluated by the absence of peaks in an X-ray powder diffractogram (XRPD).

By *"chemically stable"* it is meant that the solid form of the present invention shows no degradation upon storage under stressed conditions, e.g. when stored (I) at least 70 °C under reduced pressure for at least 1 hour (preferably for 5 hours, more preferably for 10 hours, even more preferably for 12 hours), (II) at 60 °C for at least 1 day (preferably for 5 days, more preferably for 10 days, even more preferably for 15 days), (III) at 40 °C and 75% RH for at least 1 day (preferably for 5 days, more preferably for 10 days, even more preferably for 15 days), and/or (IV) at 25-30 °C and 80% RH for at least 1 day (preferably for 5 days, more preferably for 10 days, even more preferably for 15 days).

*"No degradation"* means that a HPLC analysis of the sample shows no significant worsening of the purity, i.e. without detectable formation of new impurities and without relevant increase of the content of those present in the initial profile (for example, less than 0.1 % area increase).

Unless otherwise indicated, the data relative to the peaks in the XRPD pattern are meant within the common uncertainty due to the instrument measurement, typically ± 0.2 degrees 2θ, when collected with the Kα radiation of copper (λ = 1.5418 Å). The present invention provides, in one embodiment, a polymorphically and/or chemically stable crystalline form of 2-(3,5-dichlorophenyl)-1,3-benzoxazole-6-carboxylic acid, said stable crystalline form being characterized by an XRPD profile comprising at least one of the peaks at 6.4, 11.4, 13.5, 16.3, 18.2, 20.4 and 27.5 degrees 2θ (± 0.2), when collected with the Kα radiation of copper (λ = 1.5418 Å), or a combination thereof.

Preferably, said crystalline form of 2-(3,5-dichlorophenyl)-1,3-benzoxazole-6-carboxylic acid is further characterized by an XRPD profile additionally comprising at least one of the peaks at 19.3, 22.8 and 23.5 degrees 2θ (± 0.2), more preferably 6.4 and/or 22.8, or a combination thereof.

As a non-limiting example, a full peak list of a representative form alpha XRPD pattern is provided hereunder (I/I0 = relative intensity, only peaks having relative intensity equal to or higher than 2% are indicated):

| **2theta [°]** | **d [Å]** | **I/I0** |
|---|---|---|
| 5.25 | 16.8390 | 33.11 |
| 6.07 | 14.5727 | 79.46 |
| 6.41 | 13.7958 | 295.89 |
| 6.77 | 13.0659 | 75.28 |
| 7.50 | 11.7852 | 28.93 |
| 9.62 | 9.1922 | 62.16 |

| | | |
|---|---|---|
| 11.45 | 7.7266 | 119.85 |
| 12.32 | 7.1860 | 22.14 |
| 13.53 | 6.5427 | 661.51 |
| 16.27 | 5.4489 | 384.38 |
| 18.20 | 4.8740 | 116.72 |
| 19.31 | 4.5966 | 302.81 |
| 20.36 | 4.3613 | 1000.00 |
| 21.49 | 4.1346 | 62.49 |
| 22.75 | 3.9083 | 223.09 |
| 23.52 | 3.7824 | 240.77 |
| 24.33 | 3.6580 | 104.39 |
| 24.78 | 3.5936 | 20.12 |
| 26.88 | 3.3173 | 117.24 |
| 27.47 | 3.2472 | 728.94 |
| 30.68 | 2.9146 | 72.66 |
| 31.70 | 2.8227 | 15.96 |
| 34.99 | 2.5647 | 29.78 |
| 35.53 | 2.5266 | 21.55 |

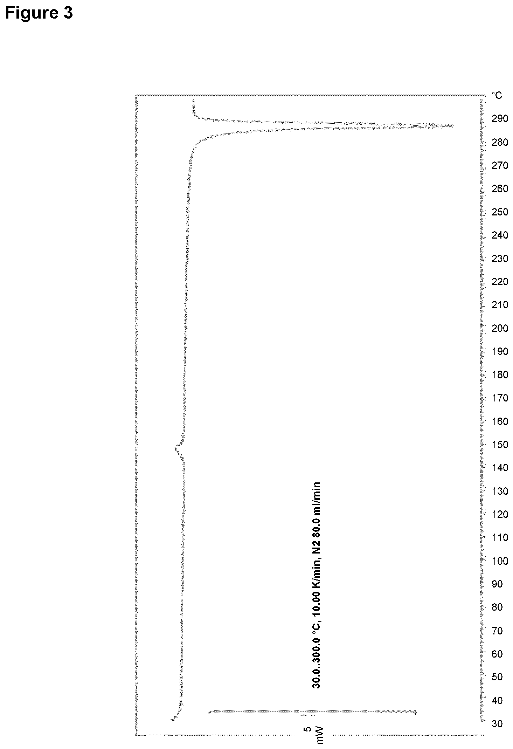

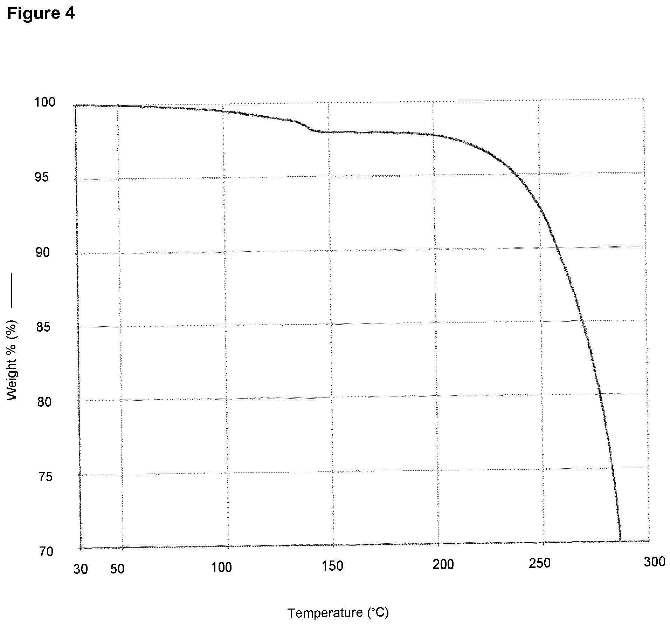

In a preferred embodiment, said crystalline form alpha is characterized by an XRPD profile comprising at least both of the peaks at 6.4 ,11.4 and 18.2 degrees 2θ (± 0.2) and, optionally, also the peak at 22.8 degrees 2θ (± 0.2). In a preferred embodiment, said crystalline form alpha is characterized by an XRPD profile comprising at least each of the peaks at 11.4, 13.6, 16.3, 18.2, 20.4 and 27.5 degrees 2θ (± 0.2). In a preferred embodiment, the present invention relates to the crystalline form alpha as described above, further characterized by a DSC profile similar to that shown in figure 3, i.e. having an endothermic transition with onset at 286 ± 1 °C and a peak at 287 ± 1 °C, and/or by a TGA profile similar to that shown in figure 4 and/or an IR spectrum having at least one of 1695, 1573, 1547, 1437, 1420, 1298, 1276, 882, 862, 772, 745, 725, 678, 665, 534 cm⁻¹.

It was found that form alpha according to the present invention is stable, in that it does not change its polymorphic form and its chemical purity does not decrease over storage at 20 - 60 °C for several weeks. In addition, form alpha of tafamidis according to the present invention can be used to prepare a finished dosage form for pharmaceutical usage or it could be advantageously converted to other crystalline forms of tafamidis (e.g. form 1 or form 4 as described in WO 2016/38500) or salts, such as the meglumine salt, in high crystalline purity, in that only one crystalline form can be detected via XRPD analysis, via methods suitable for large-scale production.

Conversely, form alpha of tafamidis according to the present invention can be prepared from solid tafamidis in the free acid form (obtained, as non-limiting example, following the procedures disclosed in Proc Natl Acad Sci USA 2012, 109(24): 9629-9634, in WO 2016/38500, *e.g.* form 1, 4 or 6, and in the references cited therein), from a solution of Tafamidis in the acid form in a suitable solvent (*e.g.* THF) or from a salt of tafamidis (such as the meglumine salt *e.g.* obtained according to WO 2013/038351), after addition of an organic or inorganic acid (*e.g.* HCl, HBr, sulfuric acid, acetic acid, formic acid and similar acids), to obtain Tafamidis in the free acid form, optionally after precipitation from a suitable solvent.

In another embodiment, the present invention relates to a process for the preparation of the crystalline form alpha of 2-(3,5-dichlorophenyl)-1,3-benzoxazole-6-carboxylic acid as described above, comprising the steps of:
i. providing a solution of 2-(3,5-dichlorophenyl)-1,3-benzoxazole-6-carboxylic acid in a solvent;
ii. adding the solution of step i. to an anti-solvent:
iii. isolating the obtained solid.

Preferably, in said process for the preparation of form alpha, the solvent used for dissolving 2-(3,5-dichlorophenyl)-1,3-benzoxazole-6-carboxylic acid in step i. is selected from the group consisting of tetrahydrofuran (THF), 2-methyltetrahydrofuran, dimethyl sulfoxide, dimethylformamide, dimethylacetamide, N-methylpyrrolidone and mixtures thereof.

Preferably, in said process for the preparation of form alpha, the anti-solvent used in step ii. is selected from the group consisting of hexane, heptane, cyclohexane, methylcyclohexane, toluene, xylene, isopropyl acetate, 1,3,5-trimethylbenzene and mixtures thereof.

In a more preferred embodiment, in said process the solvent is THF and the anti-solvent is heptane.

In a preferred embodiment, in the process according to the present invention the weight / volume ratio of tafamidis / solvent is from 1 : 5 to 1 : 30, more preferably from 1 : 10 to 1 : 25 or from 1 : 15 to 1 : 20.

In a preferred embodiment, in the process according to the present invention the volume / volume ratio of solvent / antisolvent is from 1 : 1 to 1 : 5, preferably from 1 : 2 to 1 : 4 or from 1 : 2.5 to 1 : 1.3.

In a preferred embodiment, step i. in the process according to the present invention is carried out in the range from 30 to 80 °C, more preferably from 40 to 70 °C or from 50 to 60 °C.

In a preferred embodiment, step ii. in the process according to the present invention is carried out at from -30 to 25 °C, more preferably from -20 to 20 °C or from -10 to 15 °C.

In an embodiment, the present invention relates to the use of the crystalline form alpha of 2-(3,5-dichlorophenyl)-1,3-benzoxazole-6-carboxylic acid as defined above for the preparation of a solid form of 2-(3,5-dichlorophenyl)-1,3-benzoxazole-6-carboxylic acid other than form alpha, or of a salt or adduct thereof.

Preferably, the present invention relates to the preparation of form 1 or 4 of 2-(3,5-dichlorophenyl)-1,3-benzoxazole-6-carboxylic acid from solid form alpha. As non-limiting examples, form 1 and 4 of tafamidis can be prepared in high purity, i.e. free from other forms, starting from solid form alpha by dissolution/ precipitation in a suitable solvent or mixture of solvents or by heating form alpha from 60 to 160 °C, preferably from 80 to 140 °C, more preferably from 90 to 120 °C at atmospheric pressure (about 1 atm) or under vacuum.

It was found that it is possible to prepare crystalline form 1 or crystalline form 4 of tafamidis with very high polymorphic purity (*i.e.* without detectable presence of other crystalline forms via XRPD) starting from the crystalline form alpha according to the present invention via methods that are reproducible and amenable to be scaled up as industrial processes. By contrast, it was found that solid tafamidis obtained by reproducing the methods of the prior art (e.g. examples 16-20 of WO 2020/232325) on multigram scale contained detectable amounts of crystalline forms other than form 4 and that solid tafamidis obtained by reproducing the methods of example 1 of WO 2016/38500 on multigram scale contained detectable amounts of crystalline forms other than form 1.

In a preferred embodiment, the present invention relates to the preparation of a salt of tafamidis, preferably the meglumine salt of tafamidis, starting from solid form alpha of tafamidis.

In an embodiment, the present invention provides a pharmaceutical formulation comprising the crystalline form alpha of 2-(3,5-dichlorophenyl)-1,3-benzoxazole-6-carboxylic acid and, optionally, at least one excipient.

The following examples are provided to illustrate specific embodiments of the present invention, without intention to limit its scope.

The instruments and methods used to characterize the crystals obtained in the examples are as follows:
X-ray powder diffraction analyses (XRPD) were performed on a Bruker D8 Advance X-ray powder diffractometer at about 25 °C and ambient humidity (e.g. 25-35%), using a Cu Kα tube (40 kV, 40 mA, λ= 1.5418 Å) as the X-ray source, equipped with a linear Lynxeye XE-T position sensitive detector set at 250 mm from the sample. A Nickel filter (0.0125 mm thickness) was mounted on the primary beam. Data collection was made in coupled mode, theta-theta configuration over an angular range between at least 3° 2 theta and 40° 2theta and a scan step of 0.02°. Fine powder samples were placed in a flat, thin layer in the 12 mm x 0.2 mm cavity of a silicon low background plate fixed on a sample holder fitting an autosampler position. The instrument was previously calibrated by means of NIST SRM 1976b. Data acquisition was performed by means of Bruker Diffraction Measurement Center software; data elaboration was performed by means of Crystal Impact Match! or Bruker Diffrac.EVA software.

Differential scan calorimetry (DSC): DSC tests were conducted by use of a Mettler-Toledo DSC1 Stare System. Indium was used for calibration. Accurately weighed samples (3-5 mg) were placed in open aluminum vented pans and heated at a rate of 10 °C/min under 80 mL/min nitrogen purge. Range from 30 °C up to 300 °C was investigated.

Fourier transform Infrared analysis (FT-IR): FT-IR analyses were performed with a Spectrum Two FTIR Spectrophotometer equipped with a universal Attenuated Total Reflectance (ATR) and a Spectrum 10^{™} Software. Measurements were performed by carrying out 16 scans with a resolution of 4.0 cm⁻¹ in a range between 4000 and 450 cm⁻¹.

### Example 1

### Preparation of Tafamidis form alpha.

Tafamidis (10 g) was dissolved in tetrahydrofuran (220 mL) at 60-65 °C. The resulting solution was filtered through a diatomaceous earth (Hyflo^{®}) bed. Filtrate was heated to 60-65 °C and the obtained clear solution was added dropwise to heptane (730 mL) cooled to -2/+2 °C. The resulting suspension was maintained under stirring at the same temperature for 1-2 hours and then filtered. Solid was dried under vacuum at 45-50 °C for 16 hours and analyzed by XRPD.

Tafamidis form alpha was obtained.
Yield: 8.5 g

### Example 2

### Preparation of Tafamidis Form 4

Tafamidis form alpha (8.5 g) obtained according to the procedure in example 1 was dried under vacuum at 120 °C for 2 hours. The thus obtained solid was analyzed by XRPD.

Tafamidis was obtained in pure form 4, i.e. without detectable traces of other crystalline forms (e.g. by XRPD analysis).
Yield: 8.5 g

### Example 3

### Preparation of Tafamidis Form 1

Tafamidis form alpha (3 g) was suspended in 1,3,5-trimethylbenzene (mesitylene) (30 mL) and heated to 130-135 °C. Resulting suspension was maintained under stirring at the same temperature for 17 hours, then cooled to 20-25°C and filtered. Solid was washed with mesitylene (5 mL), dried under vacuum at 45-50 °C for 16 hours and analysed by PXRD.

Tafamidis Form 1 was obtained.
Yield: 3 g

### Example 4

### Preparation of Tafamidis Meglumine (form M)

Tafamidis form alpha (10 g) was suspended in a mixture of solvents isopropyl alcohol/water 5:1 (300 mL) at 20-25°C. Meglumine (6.8 g, 1.07 eq.) was added and resulting suspension was dissolved at 80°C. The obtained solution was slowly cooled to 10-15°C. Resulting suspension was maintained under stirring at the same temperature for 1-2 hours and then filtered. Solid was washed with the same mixture of solvents (15 mL), dried under vacuum at 45-50 °C for 16 hours and analysed by PXRD.

Tafamidis meglumine was obtained in crystalline Form M.
Yield: 14.9 g

## Claims

1. A polymorphically and/or chemically stable crystalline form of 2-(3,5-dichlorophenyl)-1,3-benzoxazole-6-carboxylic acid, said stable crystalline form being **characterized by** an XRPD profile comprising at least one of the peaks at 6.4, 11.4, 13.5, 16.3, 18.2, 20.4 and 27.5 degrees 2θ (± 0.2), when collected with the Kα radiation of copper (λ = 1.5418 Å), or a combination thereof.

2. The crystalline form of 2-(3,5-dichlorophenyl)-1,3-benzoxazole-6-carboxylic acid according to claim 1, **characterized by** an XRPD profile additionally comprising at least one of the peaks at 19.3, 22.8 and 23.5 degrees 2θ (± 0.2), or a combination thereof.

3. The crystalline form of 2-(3,5-dichlorophenyl)-1,3-benzoxazole-6-carboxylic acid according to any one of claims 1 and 2, being **characterized by** an XRPD profile comprising at least each of the peaks at 6.4, 11.5, 18.5 and 20.4 degrees 2θ (± 0.2).

4. The crystalline form of 2-(3,5-dichlorophenyl)-1,3-benzoxazole-6-carboxylic acid according to any one of the preceding claims, being **characterized by** a DSC profile represented in figure 3.

5. The crystalline form of 2-(3,5-dichlorophenyl)-1,3-benzoxazole-6-carboxylic acid according to any one of the preceding claims, being **characterized by** a TGA profile represented in figure 4.

6. A process for the preparation of the crystalline form of 2-(3,5-dichlorophenyl)-1,3-benzoxazole-6-carboxylic acid according to any one of the preceding claims, comprising the steps of:
i. providing a solution of 2-(3,5-dichlorophenyl)-1,3-benzoxazole-6-carboxylic acid in a solvent;
ii. adding the solution of step i. to an anti-solvent:
iii. isolating the obtained solid.

7. The process according to claim 6, wherein the solvent used for dissolving 2-(3,5-dichlorophenyl)-1,3-benzoxazole-6-carboxylic acid in step i. is selected from the group consisting of tetrahydrofuran, 2-methyltetrahydrofuran, dimethyl sulfoxide, dimethylformamide, dimethylacetamide, N-methylpyrrolidone and mixtures thereof.

8. The process according to claim 6 or 7, wherein the anti-solvent used in step ii. is selected from the group consisting of hexane, heptane, cyclohexane, methylcyclohexane, toluene, xylene, isopropyl acetate, 1,3,5-trimethylbenzene and mixtures thereof.

9. Use of the crystalline form of 2-(3,5-dichlorophenyl)-1,3-benzoxazole-6-carboxylic acid according to any one of claims 1-5 for the preparation of a solid form of 2-(3,5-dichlorophenyl)-1,3-benzoxazole-6-carboxylic acid other than form alpha, or of a salt or adduct thereof.

10. The use according to claim 9 for the preparation of form 1 or form 4 of 2-(3,5-dichlorophenyl)-1,3-benzoxazole-6-carboxylic acid.

11. The use according to claim 9 for the preparation of the meglumine salt of 2-(3,5-dichlorophenyl)-1,3-benzoxazole-6-carboxylic acid.

12. A pharmaceutical formulation comprising the crystalline form of 2-(3,5-dichlorophenyl)-1,3-benzoxazole-6-carboxylic acid according to claims 1-5.
